# EUROPÄISCHE PATENTSCHRIFT

(11) **EP 3 518 801 B1**
(45) Veröffentlichungstag und Bekanntmachung des Hinweises auf die Patenterteilung: **04.11.2020**
(21) Anmeldenummer: 17784219.2
(22) Anmeldetag: 29.09.2017
(51) Int. Cl.: A61B 18/22, A61B 90/90

(54) **SYSTEM ZUR CHIRURGISCHEN BEHANDLUNG**
SYSTEM FOR SURGICAL TREATMENT
SYSTÈME POUR UN TRAITEMENT CHIRURGICAL

(30) Priorität: 30.09.2016 DE 102016118663
(43) Veröffentlichungstag der Anmeldung: 07.08.2019
(73) Patentinhaber: IMS GmbH, 82327 Tutzing (DE)
(72) Erfinder: SCHUBERT, Michael, 82327 Tutzing/Traubing (DE)
(74) Vertreter: Soria Parra, Manuel
(86) Internationale Anmeldenummer: PCT/EP2017/074783
(87) Internationale Veröffentlichungsnummer: WO 2018/060429

(56) Entgegenhaltungen:
- EP-A1- 1 410 766
- WO-A1-2014/126558
- DE-A1-102008 044 977
- US-A1- 2015 272 654

## Beschreibung

Die Erfindung betrifft ein System zur chirurgischen Behandlung, insbesondere zur endovenösen Lasertherapie, nach dem Oberbegriff des Patentanspruchs 1. Ein derartiges System ist beispielsweise aus DE 10 2008 044 977 A1 bekannt.

Das bekannte System umfasst ein multifunktionales Lasergerät, welches eine Laserdiode aufweist, die Laserlicht mit einer Wellenlänge von 1920 nm emittieren kann. Das Laserlicht wird über eine Lichtleitfaser appliziert, wobei eine Applikation insbesondere im Bereich der Urologie, der Gastroenterologie und der HNO vorgesehen ist.

DE 20 2005 022 114 U1 beschreibt ein System zur chirurgischen Behandlung, das eine lichtemittierende Vorrichtung mit einer Anregungslichtquelle und einem Wellenlängenumwandlungselement sowie einen Katheter bzw. ein Endoskop umfasst, das mit der lichtemittierenden Vorrichtung verbindbar ist. Das Endoskop weist einen Lichtwellenleiter auf, der optisch mit der lichtemittierenden Vorrichtung gekoppelt wird, um Licht über den Katheter an den Behandlungsort zu leiten. Die Lichtquelle kann ein Laserdiodenelement mit einem Nitridhalbleiter umfassen, das Licht in einem Wellenlängenbereich zwischen 350 nm und 500 nm, vorzugsweise zwischen 420 nm und 490 nm, emittiert. Solche Wellenlängen sind geeignet, um den Behandlungsort auszuleuchten, bewirken jedoch keine direkte Behandlung von Gewebe. Zur chirurgischen Behandlung wird bei dem vorbekannten Gerät vielmehr ein separates Instrument über den Katheter bzw. das Endoskop an den Behandlungsort geführt.

Ein System zur chirurgischen Behandlung von Gewebe mittels Laserlicht ist aus US 2009/0248004 A1 bekannt. Das System umfasst ein Lasergerät und ein Handstück, das über einen Lichtwellenleiter mit dem Lasergerät verbindbar ist. Das Handstück kann auf ein Gewebeareal gerichtet werden, so dass Laserlicht, welches im Lasergerät erzeugt wird, zur Koagulation von Gewebe eingesetzt werden kann. Das bekannte Gerät nutzt als Laserlichtquelle entweder LEDs auf Basis eines Nitridhalbleiters oder Laserdioden. Für die Behandlung von Venen werden Wellenlängen im Bereich von 600 nm bis 1.500 nm vorgeschlagen. Nachteilig bei der bekannten Vorrichtung ist die Verwendung einer Wellenlänge von kleiner 1800 nm, was eine hohe Leistungsdichte der Laserlichtquelle erfordert. Das emittierte Laserlicht dringt dabei tiefer in die Gewebestrukturen ein und kann so tieferliegende Gewebestrukturen in unerwünschter Weise schädigen. Aufgrund des höheren Leistungsdichtebedarfs der bekannten Vorrichtung ist außerdem die Baugröße des Lasergeräts erhöht, was eine stationäre Behandlung erfordert. Gleichzeitig bringt die Behandlung einen invasiven Eingriff mit sich, da ein direkter Zugang zu den Venen bestehen muss.

WO 2014/126558 A1 und EP1410766 A1 offenbaren ein medizinisches Lasersystem mit einem Lasergerät und einer Lichtleitfaser, wobei RFID-Technologie genutzt wird, um sicherzustellen, dass bestimmte Parameter des Geräts nur beim Anschluss einer passenden Lichtleitfaser freigeschalten werden. Damit ist jedoch nicht dem Problem begegnet, dass die Lichtleitfaser mehrfach an verschiedenen Geräten eingesetzt wird, was insbesondere bei minimalinvasiven chirurgischen Eingriffen zur Übertragung von Krankheitserregern und schwerwiegenden Folgen für Patienten führen kann.

Die Aufgabe der Erfindung besteht darin, ein System zur chirurgischen Behandlung anzugeben, das kompakt aufgebaut, einfach handhabbar und kostengünstig herstellbar sowie patientensicher einsetzbar ist.

Erfindungsgemäß wird diese Aufgabe durch den Gegenstand des Patentanspruchs 1 gelöst.

Die Erfindung schlägt insoweit ein System zur chirurgischen Behandlung, insbesondere zur endovenösen Lasertherapie, vor, das ein Lasergerät und ein Applikationsmodul aufweist. Das Lasergerät umfasst eine Laserlichtquelle mit wenigstens einem ersten Laserdiodenelement. Das Applikationsmodul ist mit der Laserlichtquelle optisch verbindbar oder verbunden. Bei der Erfindung ist insbesondere vorgesehen, dass das Laserdiodenelement wenigstens eine Halbleiterschicht aus einer Antimonverbindung aufweist und so konfiguriert ist, dass Laserlicht mit einer ersten Wellenlänge zwischen 1.800 nm und 2.000 nm, insbesondere mit einer Wellenlänge von 1.940 nm, erzeugbar ist. Das Lasergerät kann so eingestellt sein, dass kontinuierliches oder gepulstes Laserlicht mit den zuvor genannten Wellenlängen erzeugbar ist bzw. erzeugt wird.

Bei der Erfindung wird durch die Verwendung einer Halbleiterschicht aus einer Antimonverbindung ein Halbleiterdiodenlaser bereitgestellt, der Laserlicht in einem Wellenlängenbereich zwischen 1.800 nm und 2.000 nm mit einem hohen elektrisch-optischen Wirkungsgrad direkt emittiert. Auf einen aufwändigen Pumpmechanismus, wie er beispielsweise bei Thulium-YAG-Lasern, bekannt ist, kann daher verzichtet werden. Das führt dazu, dass das Lasergerät des erfindungsgemäßen Systems besonders kompakt aufgebaut sein kann.

Erfindungsgemäß ist vorgesehen, das Lasergerät im Zusammenhang mit einem Applikationsmodul zu nutzen, das als Katheter ausgebildet ist. Die Verwendung eines Katheters ermöglicht eine minimalinvasive chirurgische Behandlung. So können beispielsweise endovenöse Therapien einfach und für den Patienten schonend durchgeführt werden. Im Allgemeinen hat es sich für die Laserlichtbehandlung über ein beliebiges Applikationsmodul als besonders vorteilhaft erwiesen, eine Wellenlänge zwischen 1.800 nm und 2.000 nm einzustellen. Bei diesen Wellenlängen kann mit geringen Leistungsdichten eine hohe chirurgische Therapieleistung erbracht werden.

Die bei der Erfindung gewählte Wellenlänge hat insofern mehrere Vorteile. Einerseits wird damit eine gute Behandlung verschiedener Gewebearten des menschlichen Körpers erreicht, da die vorgeschlagene Wellenlänge aufgrund des hohen Wasseranteils des Gewebes gut absorbierbar ist. Gleichzeitig wird bei der vorgeschlagenen Wellenlänge erreicht, dass eine Lichtübertragung mittels kostengünstiger Glas-Lichtwellenleiter weitgehend verlustfrei möglich ist. Im Ergebnis sind daher geringere Leistungen der Laserlichtquelle erforderlich, um im Vergleich zu anderen Wellenlängen, die bislang im Stand der Technik eingesetzt werden, dieselbe Therapieleistung zu erbringen. Insbesondere im Vergleich zu den Wellenlängen, die bei dem System gemäß der eingangs genannten US 2009/0248004 A1 vorgeschlagen werden, wird mit der vorliegenden Erfindung die für denselben Therapieerfolg erforderliche Laserlichtleistung mindestens halbiert.

Die vorgeschlagene Wellenlänge hat außerdem Vorteile hinsichtlich der Behandlung des Gewebes, da durch die höhere Absorptionsrate des Laserlichts im Gewebe die Eindringtiefe des Laserlichts begrenzt ist. So kann effizient verhindert werden, dass tieferliegende, für den Anwender meist nicht sichtbare Gewebeschichten, unbeabsichtigt beeinträchtigt werden. Insofern eignet sich das erfindungsgemäße System insbesondere zur Behandlung dünner Gewebeschichten, beispielsweise zur Behandlung von Venenwänden, die in der Regel eine dünne Wandstärke aufweisen. Im Ergebnis wird die chirurgische Behandlung somit sehr schonend für den Patienten durchgeführt, so dass der Heilungsprozess insgesamt beschleunigt wird. Damit geht auch eine verbesserte Präzision der Laserbehandlung einher.

Die Nutzung geringerer Laserlichtleistungen zur Erzielung des gewünschten chirurgischen Effekts führt auch zu dem besonderen Vorteil, dass das Lasergerät des erfindungsgemäßen Systems besonders kompakt gestaltet werden kann. Da die Laserlichtquelle eine geringe Leistungsaufnahme benötigt, kann die Kühlung der Laserlichtquelle durch Luft erfolgen. Insofern ist es im Rahmen der Erfindung bevorzugt vorgesehen, wenn das Lasergerät eine luftgekühlte Laserlichtquelle aufweist. Ein aufwändiger Kühlmittelkreislauf, der im Regelfall einen großen Bauraumbedarf hat, wird auf diese Weise vermieden. Insgesamt können so die Außenabmessungen des Lasergeräts reduziert werden, was mobile Anwendungen des Lasergeräts ermöglicht.

Bei der bevorzugten Variante der Erfindung ist vorgesehen, dass die Halbleiterschicht des Laserdiodenelements eine Galliumantimonidverbindung aufweist oder aus einer Galliumantimonidverbindung besteht. Es hat sich gezeigt, dass eine solche Halbleiterschicht, insbesondere eine Galliumantimonid-Halbleiterschicht, besonders effizient eingesetzt werden kann, um Laserlicht direkt zu erzeugen, welches eine ausreichende Leistungsdichte aufweist, um für die chirurgische Behandlung eingesetzt zu werden. Dabei ist insbesondere vorgesehen, dass die Laserlichtquelle direkt abstrahlende EinzelemitterLaserdioden aufweist. Insbesondere können mehrere Laserdiodenelemente vorgesehen sind, die zu einem Array verschaltet sind. Um eine möglichst hohe Leistungsdichte zu erreichen, können die kollimierten Strahlen der EinzelemitterLaserdioden dicht gepackt werden, insbesondere in einer hexagonalen Linsenstruktur. Diese Hexagonal-Anordnung stellt die größte Packungsdichte zur Erreichung einer quasi-rotationssymmetrischen Summen-Strahlform dar, was wiederum für eine maximale Einkopplungs-Effizienz in runde Applikationsfasern (Katheter) zweckmäßig ist. Die mögliche Verwendung von EinzelemitterLaserdioden, beispielsweise in Form von Breitstreifen-Laserdioden, bewirkt eine Verbesserung der Strahlqualität im Vergleich zu Barren-Laserdioden.

Generell können mehrere erste Laserdiodenelemente vorgesehen sein, die Laserlicht mit einer ersten Wellenlänge zwischen 1.800 nm und 2.200 nm erzeugen. Ergänzend kann vorgesehen sein, dass die Laserlichtquelle wenigstens ein zweites Laserdiodenelement aufweist, das Laserlicht mit einer zweiten Wellenlänge emittiert. Es können auch mehrere zweite oder weitere (dritte, vierte usw.) Laserdiodenelemente vorgesehen sein. Die zweite Wellenlänge kann von der ersten Wellenlänge verschieden sein.

Das wenigstens eine zweite Laserdiodenelement ist vorzugsweise als Einzelemitter-Laserdiode oder als Breitstreifen-Laserdiode ausgebildet und kann Bestandteil desselben Laserdiodenmoduls sein, das auch das wenigstens eine erste Laserdiodenelement umfasst. Es ist auch möglich, dass das zweite Laserdiodenelement optisch mit dem Laserdiodenmodul gekoppelt ist, welches das erste Laserdiodenelement umfasst.

Die Kombination von mehreren Laserdiodenelementen, die unterschiedliche Wellenlängen emittieren, hat den Vorteil, dass die Einsatzmöglichkeiten des erfindungsgemäßen Systems erhöht werden. So können unterschiedliche Anwendungen mit demselben Lasergerät umgesetzt werden. Beispielsweise kann mit einer ersten Wellenlänge eine Koagulation von Gewebe erreicht werden, wogegen mit einer zweiten Wellenlänge Laserlicht zum Schneiden von Gewebe (Dissektion) und/oder zur Koagulation und/oder zur Ablation von Gewebe und /oder zur Ausleuchtung des Behandlungsobjekts eingesetzt wird. Andere Kombinationen von Anwendungsmöglichkeiten sind bei entsprechender Auswahl von Laserdiodenelementen unterschiedlicher Wellenlänge möglich.

Erfindungsgemäß ist vorgesehen, dass das Applikationsmodul einen Lichtwellenleiter aufweist. Der Lichtwellenleiter ist flexibel. Insbesondere bildet das Applikationsmodul einen biegeflexiblen Katheter, der den Lichtwellenleiter umfasst.

Vorzugsweise ist vorgesehen, dass das Lasergerät und/oder das Applikationsmodul eine Koppeloptik, insbesondere wenigstens eine Linse, zur Einkopplung des von der Laserlichtquelle erzeugten Laserlichts in den Lichtwellenleiter aufweist. Insbesondere wird darauf hingewiesen, dass im Rahmen der Erfindung explizit vorgesehen ist, dass die Laserlichtquelle mehrere Laserdiodenelemente mit unterschiedlicher Wellenlänge aufweist. Insofern kann vorgesehen sein, dass alle Laserdiodenelemente mit der Koppeloptik so verbunden sind, dass Laserlicht unterschiedlicher Wellenlänge in denselben Lichtwellenleiter des Applikationsmoduls einkoppelbar ist. Auf diese Weise kann über den Lichtwellenleiter des Applikationsmoduls Laserlicht mit unterschiedlicher Wellenlänge an den Behandlungsort geführt werden. Das erhöht die Flexibilität des Systems erheblich, da ohne einen Wechsel des Applikationsmoduls, insbesondere ohne einen Katheterwechsel, unterschiedliche Behandlungsformen realisierbar sind.

Konkret ist so eine hybride Applikation von Laserlicht unterschiedlicher Wellenlänge möglich, beispielsweise um eine größervolumige Koagulation einerseits und durch einfaches Umschalten der entsprechenden Laserdiodenelemente eine gering eindringende Dissektion andererseits zu ermöglichen. Bei der Erfindung ist insofern bevorzugt vorgesehen, dass das erste Laserdiodenelement und das zweite Laserdiodenelement so angeordnet sind, dass von dem ersten und dem zweiten Laserdiodenelement emittiertes Laserlicht abwechselnd und/oder gleichzeitig in den, insbesondere denselben, Lichtwellenleiter, insbesondere über die Koppeloptik, einkoppelbar ist.

Besonders vorteilhaft ist es, die Koppeloptik in das Lasergerät zu integrieren. Damit wird die Komplexität der mit dem Lasergerät kombinierbaren Applikationsmodule reduziert. Die Applikationsmodule, vorzugsweise Katheter, die oft als Einmalprodukt gestaltet sind, können auf diese Weise kostengünstig hergestellt werden.

Die Laserdiodenelemente können in elektrischer Serienschaltung miteinander verbunden sein. Das gilt einerseits für die mehreren ersten Laserdiodenelemente, die untereinander in Serienschaltung gekoppelt sein können. Andererseits können auch die mehreren zweiten Laserdiodenelemente elektrisch in Serie miteinander verschaltet sein. Durch die elektrische Serienschaltung der Laserdiodenelemente untereinander werden die elektrischen Gesamtströme innerhalb des Lasergeräts reduziert. Somit können kleinere Kabelquerschnitte eingesetzt werden, was einerseits Bauraum einspart und andererseits eine verbesserte Luftkühlung innerhalb des Lasergeräts ermöglicht.

Bei dem erfindungsgemäßen System weist das Lasergerät eine Steuerung auf, die mit der Laserlichtquelle verbunden ist. Die Steuerung kann so konfiguriert sein, dass eine gepulste und/oder kontinuierliche Laserlichtemission einstellbar ist. Vorzugsweise wird die Ansteuerung der Laserlichtquelle ausschließlich elektronisch mittels der Steuerung realisiert. Das hat den Vorteil, dass beliebige Modi der Ansteuerung eingestellt werden können. Insbesondere können unterschiedliche elektrische Pulswellenmodulationen mittels der Steuerung realisiert werden. Im gepulsten Anregungsfall kann die optische Spitzen-Ausgangsleistung der Laserdiodenelemente abhängig vom Duty-Cycle gegenüber der nominalen kontinuierlichen Ausgangsleistung signifikant überhöht werden, typischerweise um einen Faktor 2 bis 4. So kann das Lasergerät auch nachträglich an zukünftige Behandlungsmuster angepasst werden.

Bei dem erfindungsgemäßen System ist außerdem bevorzugt vorgesehen, dass die Laserlichtquelle mit einer elektrischen Betriebsgleichspannung von höchstens 50 Volt betreibbar ist. Durch die Verwendung von direkt abstrahlenden Einzelemitter-Laserdiodenelementen wird insgesamt eine hohe Energieeffizienz erreicht. Insoweit reicht es aus, die Laserlichtquelle mit einer relativ geringen Gleichspannung von 50 Volt zu versorgen. Dies hat insbesondere mit Blick auf Medizinprodukte-Normen erhebliche Vorteile. Einerseits wird durch die geringe Betriebsspannung das elektrische Gefährdungsrisiko durch das Medizinprodukt reduziert. Insbesondere sind bei niedrigen Spannungen geringe Ableitströme zu erwarten, so dass die Sicherheit für das Bedienpersonal erheblich erhöht ist.

Die geringeren Betriebsgleichspannungen, die zum Betrieb der Laserlichtquelle erforderlich sind, ermöglichen es auch, das Lasergerät mobil zu betreiben. Insofern ist besonders bevorzugt vorgesehen, wenn das Lasergerät eine Spannungsquelle, insbesondere einen Akkumulator, aufweist, der die Betriebsgleichspannung von höchstens 50 Volt bereitstellt. Niedrige Betriebsgleichspannungen können ohne weiteres durch kompakte Gleichspannungsquellen, beispielsweise Batterien oder einen Akkumulator, bereitgestellt werden. Insofern kann ein kompaktes und tragbares Lasergerät realisiert werden. Damit eignet sich das erfindungsgemäße System in dieser Variante insbesondere für den mobilen Einsatz.

Bei der Erfindung ist ferner bevorzugt vorgesehen, dass das Lasergerät ein gas- und/oder flüssigkeitsdichtes Gehäuse aufweist, wobei zumindest die Laserlichtquelle in dem Gehäuse angeordnet ist. Konkret kann vorgesehen sein, dass zumindest die Laserlichtquelle, ggf. auch weitere Komponenten des Lasergeräts, hermetisch dicht abgeschlossen ist. Dazu dient das gas- und/oder flüssigkeitsdichte Gehäuse. Auf diese Weise ist ein Betrieb des Lasergeräts auch in feuchten und/oder staubigen Umgebungen möglich. Im Zusammenspiel mit der hohen Mobilität, die durch das kompakte Lasergerät bereitgestellt wird, ergeben sich insofern weitere Anwendungsbereiche. Um die Gas- und/oder Flüssigkeitsdichtigkeit des Gehäuses bzw. des Lasergeräts insgesamt zu erreichen, kann zusätzlich vorgesehen sein, dass an einem Modulanschluss des Lasergeräts ein Schutzglas vorgesehen ist. Das Schutzglas deckt den Anschluss für das Applikationsmodul, das eine Lichtübertragung aus dem Lasergerät in das Applikationsmodul, insbesondere den Lichtwellenleiter des Katheters, ermöglicht, dichtend ab. Vorzugsweise ist das Schutzglas so am Modulanschluss des Lasergeräts angeordnet bzw. befestigt, dass es ohne Öffnen des Gehäuses des Lasergeräts austauschbar ist.

Hinsichtlich des Applikationsmoduls, insbesondere des innerhalb des Applikationsmoduls angeordneten Lichtwellenleiters, ist vorgesehen, dass eine Kopplung mit dem Lasergerät nur dann möglich ist, wenn der Lichtwellenleiter für die emittierte Wellenlänge der Laserlichtquelle geeignet ist. Um eine entsprechende Identifikation des Lichtwellenleiters des Applikationsmoduls zu ermöglichen, ist bei der Erfindung vorgesehen, dass das Applikationsmodul, insbesondere der Lichtwellenleiter, einen RFID-Chip mit einer Parameter- und /oder Freigabecodierung aufweist. Vorzugsweise ist der RFID-Chip an einem proximalen Ende des Applikationsmoduls, insbesondere des Lichtwellenleiters, angeordnet.

Die Steuerung des Lasergeräts weist eine RFID-Sender- und Empfängereinheit zum Auslesen und/oder Beschreiben des RFID-Chips auf. Dabei ist die Steuerung so konfiguriert, dass eine Parameter-Voreinstellung und/oder eine Aktivierung der Laserlichtquelle in Abhängigkeit von der Erfassung einer vorbestimmten Parameter- und/oder Freigabecodierung durch die RFID-Empfängereinheit erfolgt. Mit anderen Worten werden die zum Applikationsmodul passenden Betriebsarten des Lasergeräts nur freigeschalten, wenn ein Applikationsmodul mit dem Modulanschluss des Lasergeräts verbunden ist, wobei der RFID-Chip des Applikationsmoduls eine entsprechende Codierung aufweist, die als Parameter- und/oder Freigabecodierung von der RFID-Sender- und Empfängereinheit der Steuerung erkannt wird. Auf diese Weise werden Fehlbedienungen vermieden.

Außerdem können während der Behandlung die verwendeten Laserparameter, insbesondere die applizierte Gesamt-Laserenergie oder die Anzahl der Verwendungen des jeweiligen Applikationsmoduls, auf den RFID-Chip zurückgeschrieben werden. Konkret ist vorgesehen, dass die Steuerung einen Zeitstempel erstellt, der auf dem RFID-Chip gespeichert wird und die erstmalige Verwendung des Applikationsmoduls dokumentiert. Auf diese Weise können Einweg-Applikationsmodule nach ihrer Verwendung entwertet bzw. unbrauchbar gemacht werden.

Wie bereits ausführlich dargestellt wurde, zeichnet sich das Lasergerät bei dem erfindungsgemäßen System insbesondere wegen Verwendung einer Laserlichtquelle mit direkt abstrahlenden Antimonid-Halbleiter-Laserdiodenelementen, durch eine besonders kompakte Bauweise aus. Insofern ist es bevorzugt, wenn das Lasergerät ein Volumen von höchstens 10 dm³, insbesondere höchstens 9,5 dm³, aufweist. Konkret kann vorgesehen sein, dass das Lasergerät eine Höhe von höchstens 150 mm, eine Breite von höchstens 250 mm und/oder eine Tiefe von höchstens 250 mm aufweist. Das Lasergerät ist vorzugsweise so gestaltet, dass die Masse des Lasergeräts höchstens 10 kg, vorzugsweise höchstens 8 kg, beträgt. Die geringen Außendimensionen und das geringe Gewicht des Lasergeräts tragen erheblich zu den Eigenschaften des Lasergeräts bei, die eine mobile Anwendung des gesamten Systems ermöglichen.

Bei dem erfindungsgemäßen System kann außerdem vorgesehen sein, dass das Lasergerät eine Hilfslaserlichtquelle und einen Lichtsensor umfasst. Die Hilfslaserlichtquelle und der Lichtsensor können mit der Steuerung so gekoppelt sein, dass eine Laserlichtintensität der Laserlichtquelle in Abhängigkeit einer spezifischen Fluoreszenzlichtsignatur des zu behandelnden Gewebes einstellbar ist. Ziel dieser Gestaltung des Lasergeräts ist es, die Laserlichtintensität der Laserlichtquelle durch eine Regelung zu beeinflussen, wobei als Regeleingangsgröße die Fluoreszenzlichtsignatur des zu behandelnden Gewebes eingeht.

Es wurde erkannt, dass sich optische Eigenschaften wie Fluoreszenz und Reflexion von Gewebe bei der chirurgischen Behandlung des Gewebes, insbesondere bei der Koagulation, ändern. Dabei besteht ein direkter Zusammenhang zwischen der Fluoreszenzlichtsignatur und dem Koagulationsgrad des Gewebes. Die Erfindung sieht in dieser bevorzugten Variante vor, mittels einer Hilfslaserlichtquelle Laserlicht auf das zu behandelnde Gewebe zu richten, welches reflektiert und in den Lichtwellenleiter des Applikationsmoduls zurückgestrahlt wird. Über den Lichtwellenleiter des Applikationsmoduls gelangt also reflektiertes Hilfslaserlicht an den Lichtsensor. Das Hilfslaserlicht weist eine Fluoreszenzlichtsignatur auf, die vom Koagulationsgrad des behandelten Gewebes abhängig ist. Die Steuerung wertet nun die durch den Lichtsensor erfasste Fluoreszenzlichtsignatur aus und passt die Laserlichtintensität der Laserlichtquelle entsprechend an den Koagulationsgrad des behandelten Gewebes an. Insoweit ist ein geschlossener Regelkreis gebildet. Insbesondere kann eine unbeabsichtigte Schädigung von nicht zu behandelndem Gewebe effizient vermieden werden, indem die Leistung des Lasergeräts je nach Regelsignal reduziert oder abgeschaltet wird.

Eine weitere bevorzugte Ausgestaltung der Erfindung sieht vor, dass das Lasergerät einen Temperatursensor zur Erfassung einer Temperatur des zu behandelnden Gewebes aufweist. Der Temperatursensor kann mit der Steuerung so gekoppelt sein, dass eine Laserlichtintensität der Laserlichtquelle in Abhängigkeit der Gewebetemperatur einstellbar ist. Der Temperatursensor kann als optischer Sensor realisiert sein. Insofern kann der zuvor beschriebene Lichtsensor auch zur Erfassung der Temperatur des behandelten Gewebes genutzt werden. Alternativ ist es möglich, einen separaten Temperatursensor vorzusehen, der beispielsweise direkt mit dem zu behandelnden Gewebe in Kontakt gebracht wird.

Im Allgemeinen ist bei der Erfindung bevorzugt vorgesehen, dass die Steuerung eine Auswerteeinheit umfasst, die mit dem Lichtsensor und/oder dem Temperatursensor gekoppelt ist und die Temperatur des Gewebes bzw. den Koagulationsgrad des Gewebes anhand der Fluoreszenzlichtsignatur erfasst und die erfassten Parameter auswertet. Die Auswerteeinheit umfasst vorzugsweise die Hilfslaserlichtquelle. Dabei kann die Hilfslaserlichtquelle einen gepulsten Laserlichtstrahl oder einen kontinuierlichen Laserlichtstrahl aussenden. Die Auswerteeinheit kann außerdem Verstärkerfunktionen aufweisen, um das reflektierte Hilfslaserlicht zum Zwecke der Auswertung zu verstärken.

Das erfindungsgemäße System kann außerdem in einer bevorzugten Ausgestaltung eine Speichereinheit zur Speicherung von Behandlungsinformationen aufweisen. Insbesondere können Informationen über die Laserlichtleistung, die Menge der applizierten Energie und/oder die Betriebsdauer und/oder der Lichtwellenleitertyp etc. in der Speichereinheit gespeichert werden. Die gespeicherten oder zu speichernden Daten können über eine elektronische Schnittstelle, insbesondere kabellos, ausgegeben werden. Auf diese Weise können einfach Statistiken und Behandlungsnachweise erstellt werden, was die Dokumentation der Behandlung erleichtert.

Im Allgemeinen wird darauf hingewiesen, dass das Lasergerät mit unterschiedlichen Applikationsmodulen verbindbar ist. Insofern kann ein Set bzw. Modulbaukasten bereitgestellt werden, wobei das Lasergerät als Grundmodul mit unterschiedlichen Applikationsmodulen kombinierbar ist. Die Applikationsmodule ermöglichen unterschiedliche Behandlungsformen und unterscheiden sich insbesondere durch ihre optischen Eigenschaften voneinander.

In einer bevorzugten Ausgestaltung der Erfindung ist vorgesehen, dass das Lasergerät so kompakt und leicht ausgebildet ist, dass es stiftartig mit einer Hand geführt werden kann. Insbesondere kann das Lasergerät im Wesentlichen die Größe und Form eines Stiftes aufweisen, so dass es insbesondere als Laserskalpell einfach und einheitlich handhabbar ist. Das Lasergerät weist in dieser stiftartigen Gestaltung vorzugsweise einen integrierten Akkumulator auf, so dass es energieautark betrieben werden kann.

Die Erfindung wird im Folgenden anhand von Ausführungsbeispielen unter Bezugnahme auf die beigefügten schematischen Zeichnungen näher erläutert. Darin zeigen
- Fig. 1: eine perspektivische, teiltransparente Darstellung eines Lasergeräts für ein erfindungsgemäßes System nach einem bevorzugten Ausführungsbeispiel;
- Fig. 2: eine schematische Darstellung eines Laserdiodenarrays mit einer Koppeloptik zu Einkoppeln von Laserlicht in ein Applikationsmodul für ein erfindungsgemäßes System nach einem bevorzugten Ausführungsbeispiel;
- Fig. 3: eine schematische Darstellung eines Laserdiodenarrays mit einer Koppeloptik zu Einkoppeln von Laserlicht in ein Applikationsmodul für ein erfindungsgemäßes System nach einem weiteren Ausführungsbeispiel;
- Fig. 4a, 4b: jeweils eine Querschnittsansicht eines Lichtwellenleiters eines Applikationsmoduls eines erfindungsgemäßen Systems nach einem bevorzugten Ausführungsbeispiel;
- Fig. 5a-5e: jeweils eine Längsschnittansicht durch ein Kollimationslinsenarray für eine Koppeloptik zum Einkoppeln von Laserlicht in ein Applikationsmodul eines erfindungsgemäßen Systems nach einem bevorzugten Ausführungsbeispiel;
- Fig. 6: eine schematische Darstellung einer Steuerung eines Laserdiodenarrays mit einer Koppeloptik zu Einkoppeln von Laserlicht in ein Applikationsmodul für ein erfindungsgemäßes System nach einem bevorzugten Ausführungsbeispiel;
- Fig. 7: ein Diagramm zur Darstellung der wellenlängenabhängigen Absorptionsrate von Laserlicht in menschlichem Gewebe;
- Fig. 8: eine Längsschnittansicht durch ein erfindungsgemäßes System nach einem bevorzugten Ausführungsbeispiel, wobei das Lasergerät stiftartig ausgebildet und mit unterschiedlichen Applikationsmodulen kombinierbar ist; und
- Fig. 9a, 9b: jeweils eine Schaltungsanordnung mehrerer Laserdioden.

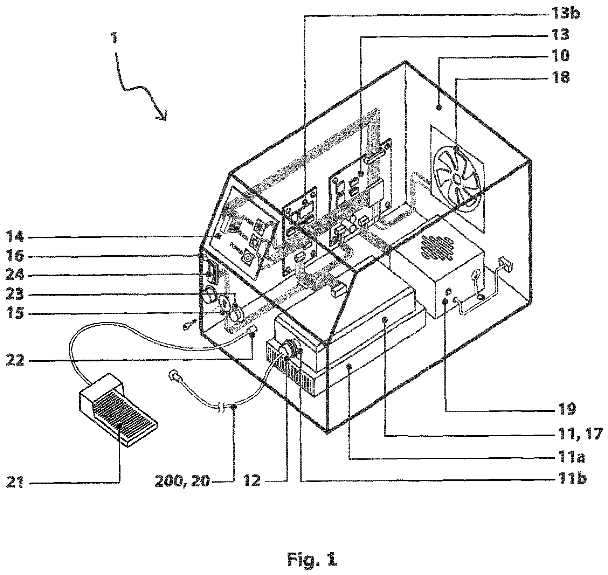

Wie in Fig. 1 erkennbar ist, weist das Lasergerät 1 ein Außengehäuse 10 auf, das die einzelnen Komponenten des Lasergeräts 1 umschließt. Innerhalb des Gehäuses ist eine Laserlichtquelle 11 angeordnet, die einen Kühlkörper 11a und eine Koppeloptik 11b aufweist. Die Koppeloptik 11b mündet in einen Modulanschluss 12, der im Gehäuse ausgebildet ist. Der Modulanschluss 12 ermöglicht die Verbindung des Lasergeräts mit einem Applikationsmodul 200, insbesondere einem Katheter 20, wobei in der beigefügten Figur aus Gründen der Übersichtlichkeit lediglich ein proximales Endstück des Katheters 20 dargestellt ist.

Der Katheter 20 weist im Wesentlichen einen Lichtwellenleiter 25 auf, der durch eine schützende Ummantelung umgeben ist. Der Lichtwellenleiter 25 ist über den Modulanschluss 12 so mit der Koppeloptik 11b optisch verbindbar, dass Laserlicht, das durch die Laserlichtquelle 11 erzeugt wird, effizient in den Lichtwellenleiter des Katheters 20 einkoppelbar ist.

Das Lasergerät 1 umfasst ferner eine Steuerung 13, die mit der Laserlichtquelle 11 verbunden ist. Die Steuerung 13 kann ferner mit einer nicht näher dargestellten Speichereinheit verbunden sein, so dass Behandlungsparameter kontinuierlich gespeichert werden können. Die Steuerung 13 ist außerdem mit Steuerelementen, insbesondere einem Touchscreen 14, gekoppelt. Ferner können Schalter vorgesehen sein, die mit der Steuerung 13 verbunden sind, um beispielsweise die Emission von Laserlicht zu aktivieren. In dem dargestellten Ausführungsbeispiel ist beispielsweise ein Schlüsselschalter 15 dargestellt, der mit der Steuerung 13 verbunden sein kann. Außerdem ist in der Zeichnung eine Leuchtanzeige 16 erkennbar, die den aktuellen Betriebszustand des Lasergeräts 1 anzeigt. Die Leuchtanzeige 16 kann beispielsweise durch eine Lampe mit einer Glühbirne oder einer LED gebildet sein.

In Fig. 1 ist ferner erkennbar, dass das Lasergerät 1 neben dem Schlüsselschalter 15 einen Notausschalter 23 aufweist. Der Notausschalter 23 ist in der üblichen Form eines Pilzkopfschalters ausgebildet. Ferner ist am Außengehäuse 10 ein Hauptschalter 24 angeordnet, der mit der Steuerung 13 verbunden ist. Zur Aktivierung der Laserlichtquelle 11 ist ferner ein Fußschalter 21 vorgesehen, der mit einem Fußschalteranschluss 22 des Lasergeräts 1 verbindbar ist.

Wie in der Zeichnung erkennbar ist, ist die Laserlichtquelle 11 in einem Gehäuse, insbesondere einem Innengehäuse 17, angeordnet. Das Innengehäuse 17 ist hermetisch abgeschlossen und unmittelbar mit dem Kühlkörper 11a verbunden. Konkret ist das Innengehäuse 17 gas- und/oder flüssigkeitsdicht ausgebildet. Ebenso ist bevorzugt vorgesehen, dass die Koppeloptik 11b in einem hermetisch abgeschlossenen Gehäuse angeordnet ist. Insbesondere kann sich das Innengehäuse 17 auch über die Koppeloptik 11b erstrecken, wobei nicht ausgeschlossen ist, dass das Innengehäuse 17 mehrteilig aufgebaut ist.

Die Steuerung 13 ist ebenfalls in einem Gehäuse angeordnet, wobei bevorzugt vorgesehen ist, dass das Gehäuse zumindest flüssigkeitsdicht geschlossen ist.

In einer Rückwand des Außengehäuses 10 ist ferner eine Lüftereinheit 18 angeordnet. Die Lüftereinheit 18 dient dazu, Luft innerhalb des Lasergeräts 1, insbesondere innerhalb des Außengehäuses 10 zu zirkulieren. Damit ist gewährleistet, dass die Rippen des Kühlkörpers ausreichend mit Luft umströmt werden, so dass eine effiziente Kühlung der Laserlichtquelle 11 erreicht wird.

Innerhalb des Außengehäuses 10 ist ferner ein Netzteil 19 angeordnet. Das Netzteil 19 ist bei dem dargestellten Ausführungsbeispiel zwischen der Laserlichtquelle 11 und der Lüftereinheit 18 vorgesehen. Eine andere Anordnung des Netzteils 19 ist möglich. Insbesondere kann ein externes Netzteil 19 zur Anwendung gelangen. Das Netzteil 19 ist mit der Steuerung 13 gekoppelt und dient als Gleichspannungsquelle für die Steuerung 13 und die Laserlichtquelle 11.

Die Laserlichtquelle 11 weist vorzugsweise mehrere Laserdiodenelemente, insbesondere mehrere Galliumantimonid-Halbleiterlaserdiodenelemente auf. Dabei können einzelne Laserdiodenelemente unterschiedliche Wellenlängen emittieren. Jedenfalls ist erkennbar, dass durch die Zusammenschaltung mehrerer einzelemittierender Halbleiterlaserdiodenelemente eine Skalierbarkeit des gesamten Systems erreicht wird. Insbesondere können je nach Anwendungsfall eine unterschiedliche Anzahl von Laserdiodenelementen zu einem Array zusammengeschaltet werden, so dass Lasergeräte mit unterschiedlichen Leistungsstärken einfach herstellbar sind.

Das in Fig. 1 dargestellte Ausführungsbeispiel eines Lasergeräts für ein erfindungsgemäßes System ist vorzugsweise so konfiguriert, dass die Laserlichtquelle 11 Laserlicht in einem Wellenlängenbereich von 1.800 nm bis 2.200 nm, vorzugsweise bei einer Wellenlänge von 1.940 nm, emittiert. Diese Wellenlänge eignet sich besonders für die endovenöse Lasertherapie. Dabei kann vorgesehen sein, dass das Lasergerät 1 so ausgelegt ist, dass die Laserlichtquelle 11 eine Laserlichtleistung bereitstellt, die an einem distalen Ende eines Lichtwellenleiters 25 des Katheters 20 eine Laserlichtleistung von höchstens 10 Watt aufweist. Vorzugsweise ist vorgesehen, dass das Laserlicht am distalen Ende des Lichtwellenleiters eine Leistung von etwa 7 Watt umfasst.

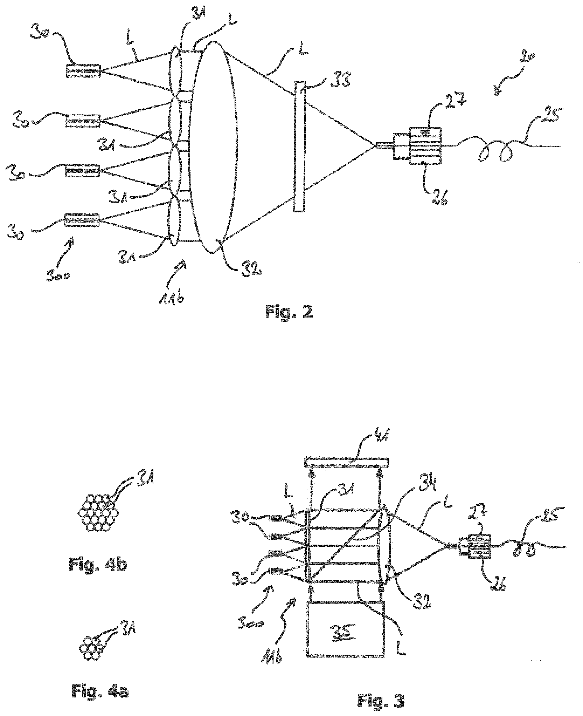

In Fig. 2 ist schematisch der Aufbau eines Arrays mit mehreren Laserdiodenelementen 30 gezeigt. Die Laserdiodenelemente 30 sind vorzugsweise als Singleemitterdioden ausgebildet. Insbesondere können die Laserdiodenelemente 30 als Breitbandlaserdioden ausgebildet sein. Die Laserdiodenelemente 30 emittieren Laserlicht L, welches jeweils über eine Kollimationslinse 31 gebündelt wird. Dabei ist jedem Laserdiodenelement 30 eine Kollimationslinse 31 zugeordnet. Das gebündelte Laserlicht gelangt nachfolgend in eine Einkoppellinse 32. Die Einkoppellinse 32 empfängt das gebündelte Laserlicht aller Laserdiodenelemente 30 und koppelt die gesamte Laserlichtmenge in den Lichtwellenleiter 25 ein. Der Lichtwellenleiter 25 weist dazu an einem proximalen Ende einen Faserstecker 26 auf, der mit dem Modulanschluss 12 des Lasergeräts 1 verbindbar ist.

Das Lasergerät 1 weist, vorzugsweise im Außengehäuse 10, ein Schutzglas 33 auf. Das Schutzglas 33 schützt die Koppeloptik 11b, die durch die Kollimationslinsen 31 und die Einkoppellinse 32 gebildet ist, vor Verschmutzung oder sonstiger Verunreinigung.

In Fig. 2 ist außerdem erkennbar, dass der Lichtwellenleiter 25, der im Allgemeinen Bestandteil eines Applikationsmoduls 200 ist, an dem Faserstecker 26 mit einem RFID-Chip 27 versehen ist. Der RFID-Chip 27 kommuniziert im Betrieb mit der Steuerung 13 des Lasergeräts 1. Insbesondere kann der RFID-Chip 27 Daten über Parameter des eingesetzten Applikationsmoduls 200, insbesondere des Lichtwellenleiters 25, aufweisen. Der RFID-Chip 27 ist außerdem beschreibbar, so dass mittels der Steuerung 13 Daten auf den RFID-Chip 27 gespeichert werden können. Konkret können Zeitstempeldaten gespeichert werden, so dass nach einer vorbestimmten Anzahl an Anwendungen das Applikationsmodul 200, insbesondere der Lichtwellenleiter 25, nicht mehr für den Betrieb mit dem Lasergerät 1 freigeschalten ist.

Mit anderen Worten kann vorgesehen sein, dass die Steuerung einen Abgleich durchführt zwischen dem RFID-Chip des Katheters 20 und einer internen Datenbank. Sobald mittels der RFID-Empfängereinheit eine spezifische RFID-Parameter- und/oder Freigabecodierung des Katheters 20 erfasst wird, gibt die Steuerung 13 die Laserlichtquelle frei, wobei auch vorgesehen ist, dass die Steuerung 13 je nach erkannter Freigabecodierung unterschiedliche Modi der Laserlichtquelle freigibt. So kann das Lasergerät 1 beispielsweise sowohl für die Veterinärmedizin als auch die Humanmedizin eingesetzt werden, wobei bei Kopplung eines Katheters 20 für die Veterinärmedizin andere Einstellmöglichkeiten für die Emission von Laserlicht bereitgestellt werden, als bei der Kopplung eines Katheters 20 für die Humanmedizin. So kann eine Fehlbedienung effizient vermieden werden. Die Katheter 20 können im Allgemeinen Einmal-Katheter oder mehrfach verwendbare Katheter 20 umfassen.

Fig. 3 zeigt eine weitere Gestaltung der Koppeloptik 11b eines erfindungsgemäßen Systems nach einem bevorzugten Ausführungsbeispiel. Im Wesentlichen ähnelt der Aufbau gemäß Fig. 3 dem Aufbau der Koppeloptik 11b gemäß Fig. 2. Insofern sind ebenfalls mehrere Laserdiodenelemente 30 vorgesehen, welchen jeweils eine Kollimationslinse 31 zugeordnet ist. Die Kollimationslinsen 13 bündeln das Laserlicht L jedes Laserdiodenelements 30. Das Laserlicht L gelangt gebündelt an eine Einkoppellinse 32, die das Laserlicht L in die Faser 25a eines Lichtwellenleiters 25 einkoppelt. Der Lichtwellenleiter 25 weist an seinem proximalen Ende einen entsprechenden Faserstecker 26 mit einem RFID-Chip 27 auf.

Im Unterschied zu dem Ausführungsbeispiel gemäß Fig. 2 ist bei dem Ausführungsbeispiel gemäß Fig. 3 zusätzlich eine Hilfslaserlichtquelle 35 vorgesehen, die seitlich zum Strahlgang des gebündelten Laserlichts L angeordnet ist. Die Hilfslaserlichtquelle 35 emittiert Hilfslaserlicht, das zur Erkennung der Gewebestruktur des zu behandelnden Gewebes eingesetzt wird. Im Strahlgang zwischen den Kollimationslinsen 31 und der Einkoppellinse 32 ist hierzu ein Strahlteiler 34 angeordnet. Der Strahlteiler 34 lenkt das Hilfslaserlicht, welches aus der Hilfslaserlichtquelle 35 stammt, um, so dass das Hilfslaserlicht ebenfalls in die Faser 25a des Lichtwellenleiters 25 eingekoppelt wird. Der Hilfslaserlichtquelle 35 ist ein Hilfslaserlichtdetektor 41 zugeordnet, der das Hilfslaserlicht erfasst und zur Übermittlung von Daten über das Hilfslaserlicht mit der Steuerung 13 signalverbunden ist.

Um eine möglichst optimale Einkopplung und Ausnutzung des von den Laserdiodenelementen 30 emittierten Laserlichts L zu erreichen, ist es vorteilhaft, die Kollimationslinsen 31 möglichst packungsdicht zueinander anzuordnen. In den Fig. 4a und 4b ist beispielhaft eine hexagonale Packung der Kollimationslinsen 31 gezeigt, wobei bei dem Ausführungsbeispiel gemäß Fig. 4a sieben Kollimationslinsen 31 und bei dem Ausführungsbeispiel gemäß Fig. 4b 19 Kollimationslinsen vorgesehen sind. Vorzugsweise sind in dem Lasergerät 1 jeweils dieselbe Anzahl von Laserdiodenelementen 30 und Kollimationslinsen 31 vorgesehen.

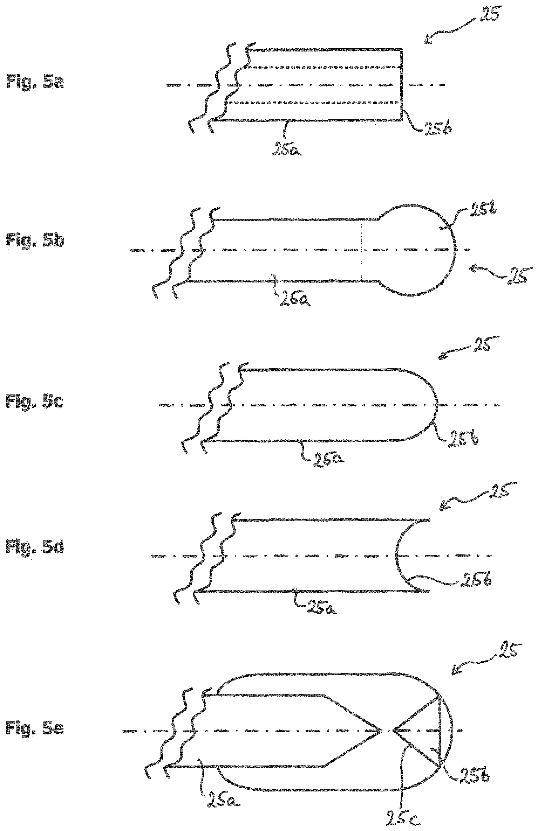

Hinsichtlich der Gestaltung des Lichtwellenleiters 25 zeigen die Fig. 5a bis 5e einige Varianten, die im Rahmen der vorliegenden Anmeldung als besonders bevorzugt angesehen werden. Für alle Lichtwellenleiter 25 gilt, dass diese eine Faser 25a aufweisen, die ein distales Faserende 25b umfasst. Über das distale Faserende 25b verlässt das Laserlicht den Lichtwellenleiter 25. Dabei kann das distale Faserelende 25b im Wesentlichen eine senkrechte Ebene zur Längsachse der Faser 25a bilden, wie dies beispielhaft in Fig. 5a dargestellt ist.

Eine besonders bevorzugte Gestaltung des distalen Faserendes 25b zeigt Fig. 5b. Das distale Faserende 25b weist hier eine ballartige bzw. kugelförmige Gestaltung auf. Eine solche Gestaltung ist insoweit bevorzugt, als dadurch eine Verletzung von Gewebe vermieden werden kann. Außerdem ist die Gestaltung des distalen Faserendes 25b gemäß Fig. 5b vorteilhaft, weil durch den vergrößerten Querschnittsdurchmesser im Bereich des distalen Faserendes 25b gegenüber der Faser 25a eine leichtere Zuführung des Lichtwellenleiters 25 durch einen Katheter erreicht wird. Insbesondere wird die Reibung zwischen dem Lichtwellenleiter 25 und der Katheterinnenwand reduziert.

Ein weiteres atraumatisches Ende zeigt beispielhaft Fig. 5c, wobei das distale Faserende 25b lediglich gekrümmt ist, ohne eine Durchschnittsüberhöhung aufzuweisen, wie dies beispielhaft in Fig. 5b gezeigt ist.

Für eine besonders konzentrierte Strahlführung kann auch ein konkaves distales Faserende 25b vorgesehen sind, wie dies in Fig. 5d gezeigt ist. Schließlich kann es in einigen Anwendungen vorteilhaft sein, das Laserlicht L radial zu emittieren. Der Lichtwellenleiter 25 gemäß Fig. 5e zeigt eine Gestaltung eines distalen Faserendes 25b mit einem Reflektor 25c, der dafür sorgt, dass durch die Faser 25a geleitetes Laserlicht L den Lichtwellenleiter 25 in radialer Abstrahlung verlässt.

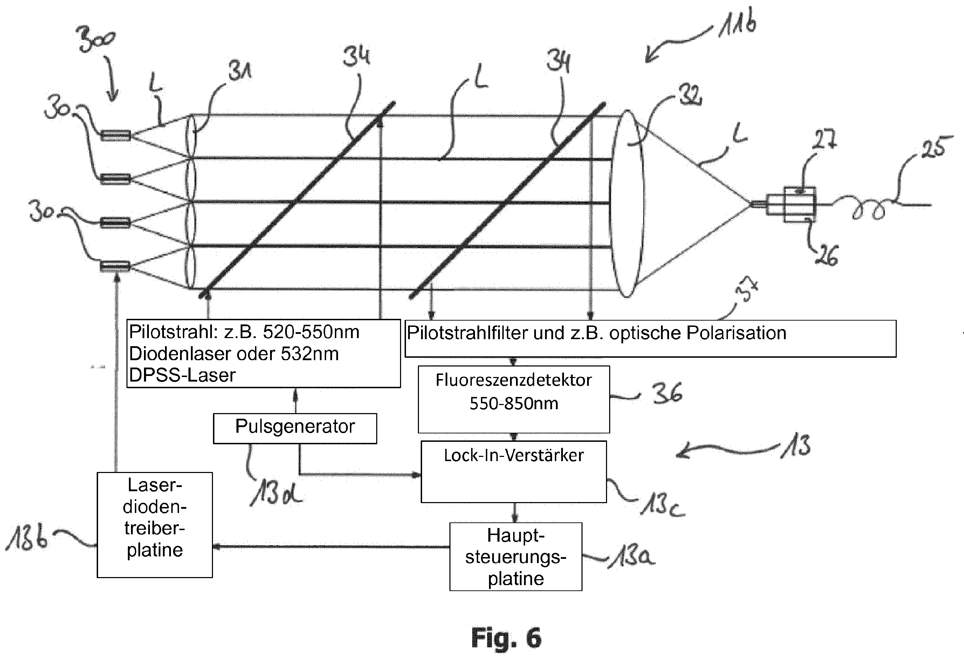

In Fig. 6 ist wiederum eine Koppeloptik 11b gezeigt, die dafür sorgt, dass Licht aus den Laserdiodenelementen 30 effizient in den Lichtwellenleiter 25 eingekoppelt wird. Die Koppeloptik 11b gemäß Fig. 6 unterscheidet sich insoweit von der Koppeloptik 11b gemäß Fig. 3, als zwei Strahlteiler 34a, 34b vorgesehen sind. Die Strahlteiler 34a, 34b sind entlang des Laserlichtstrahlengangs seriell hintereinander angeordnet. Der weitere Aufbau der Koppeloptik 11b entspricht dem Aufbau gemäß Fig. 2 und 3. Konkret sind mehrere Laserdiodenelemente 30 vorgesehen, welchen jeweils eine Kollimationslinse 31 zugeordnet ist. Zwischen dem Array aus mehreren Kollimationslinsen 31, die vorzugsweise hexagonal angeordnet sind, und einer Einkoppellinse 32 sind die beiden Strahlteiler 34 vorgesehen. In Richtung des Strahlengangs des Laserlichts L folgt auf die Einkoppellinse 32 ein Faserstecker 26, der an einem proximalen Ende eines Lichtwellenleiters 25 vorgesehen ist. Das Laserlicht L wird mittels der Einkoppellinse 32 in den Lichtwellenleiter 25, insbesondere in dessen Faser 25a, eingekoppelt. Der Faserstecker 26 kann einen RFID-Chip 27 tragen.

Im Detail ist in Fig. 6 die Steuerung der Laserlichtquelle 11 und einer Hilfslaserlichtquelle 35 gezeigt. Die Steuerung weist dazu eine Hauptsteuerung 13a auf, die elektrisch mit einem Laserdiodentreiber 13b gekoppelt ist. Der Laserdiodentreiber 13b wirkt auf die Laserdiodenelemente 30 ein und sorgt für eine entsprechende elektrische Ansteuerung der Laserdiodenelemente 30. Die Hauptsteuerung 13a erhält ferner Daten eines Verstärkers 13c, der wiederum mit einem Pulsgenerator 13d und einem Fluoreszenzlichtdetektor 36 gekoppelt ist. Der Pulsgenerator 13d ist einer Hilfslaserlichtquelle 35 zugeordnet, die Hilfslaserlicht über einen ersten Strahlteiler 34a in den Strahlengang des Laserlichts L leitet. Der erste Stahlteiler 34a ist vorzugsweise so konfiguriert, dass er für das Hilfslaserlicht kaum durchlässig ist, sondern vielmehr reflektierend wirkt. Insbesondere kann der erste Strahlteiler 34a für einen Wellenlängenbereich von 520 nm bis 550 nm hochreflektierend ausgebildet sein. Der zweite Strahlteiler 34b ist hingegen für eine Wellenlänge hochreflektierend ausgebildet, die für rückgekoppeltes Fluoreszenzlicht zu erwarten ist. Insbesondere kann der zweite Strahlteiler 34b für einen Wellenlängenbereich von 550 nm bis 850 nm hochreflektierend ausgebildet sein. Beide Strahlteiler 34a, 34b sind vorzugsweise für eine Wellenlänge im Bereich von 1.800 nm bis 2.200 nm, insbesondere 1940 nm, hochtransmittierend, so dass das Behandlungslaserlicht im Wesentlichen ungehindert passieren kann.

Der Fluoreszenzlichtdetektor 36 ist einem optischen Filter 37 nachgeordnet. Fluoreszenzlicht, welches über den Lichtwellenleiter 25 in das Lasergerät 1 zurückgekoppelt wird, gelangt an den zweiten Strahlteiler 34b, wird von dem zweiten Strahlteiler 34b umgelenkt und über den optischen Filter 37 an dem Fluoreszenzlichtdetektor 36 geleitet. Die Parameter, insbesondere die Wellenlänge, des Fluoreszenzlichts kann anschließend in der Steuerung 13 ausgewertet und kann dann genutzt werden, um den Laserdiodentreiber 13b und/oder den Pulsgenerator 13d entsprechend anzusteuern.

Das hier beschriebene Lasergerät 1 arbeitet vorzugsweise mit einer Wellenlänge im Bereich von 1800 nm bis 2000 nm, besonders bevorzugt bei einer Wellenlänge von 1940 nm. Dazu kommen vorzugsweise Laserdiodenelemente 30 zum Einsatz, die einen Halbleiter mit wenigstens einer Antimon umfassenden Halbleiterschicht aufweisen. Insbesondere kann die Halbleiterschicht eine Galliumantimonid-Verbindung umfassen.

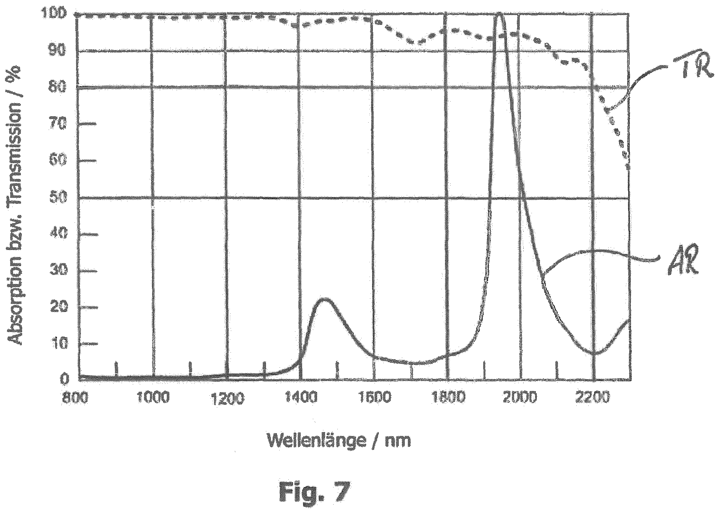

Fig. 7 zeigt deutlich, weshalb der hier bevorzugte Wellenlängenbereich für die Behandlung von Gewebe besonders vorteilhaft ist. In dem Diagramm gemäß Fig. 7 ist entlang der Abszisse die Wellenlänge in Nanometern aufgetragen. Entlang der Ordinate sind die Absorptionsrate AR menschlichen Gewebes und die Transmissionsrate TR einer Glasfaser dargestellt. Dabei sind die Werte entlang der Ordinate jeweils prozentual normiert.

Das Diagramm gemäß Fig. 7 lässt erkennen, dass die Absorptionsrate von menschlichem Gewebe im Bereich zwischen 1800 und 2200 nm deutlich ansteigt. Das bedeutet, dass Laserlicht bei einer solchen Wellenlänge einen besonders guten Effekt zur Behandlung von menschlichem Gewebe aufweist. Gleichzeitig ist die Transmissionsrate TR einer Glasfaser innerhalb dieses Wellenlängenbereichs kaum beeinträchtigt, so dass insgesamt niedrige Leistungen notwendig sind, um einen hohen Effekt im Gewebe zu erreichen. Erst bei Wellenlängen von mehr als 2200 nm sinkt die Transmissionsrate TR stark ab. Daraus ist gut erkennbar, dass das hier beschriebene Lasergerät 1 bei vergleichsweise geringer Leistungsaufnahme eine hohe Effizienz aufweist.

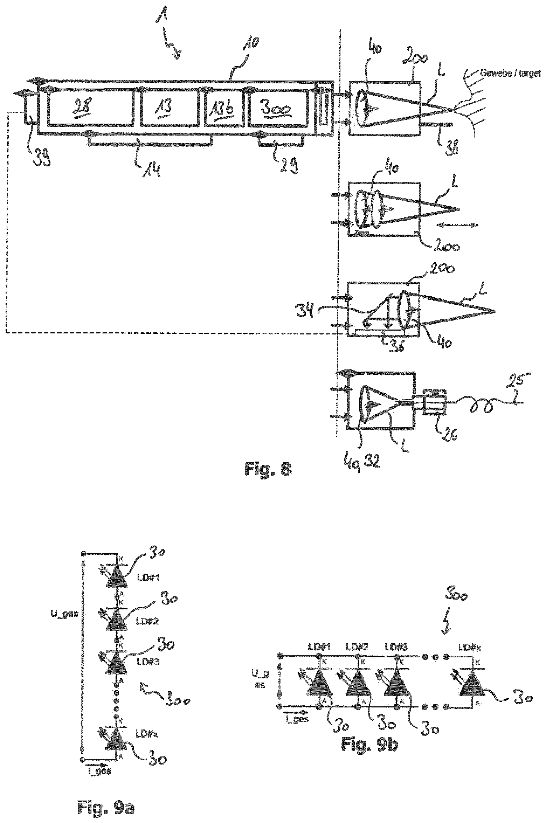

Die elektrische Leistung bzw. Leistungsaufnahme des Lasergeräts 1 steht im Allgemeinen im Zusammenhang mit dem Strom, der von den Laserdiodenelementen 30 aufgenommen wird. Besonders bevorzugt ist es, wenn Laserdiodenelemente 30 eingesetzt werden, die als Einzelemitterlaserdioden ausgebildet sind. Diese Einzelemitterlaserdioden können seriell hintereinander geschaltet werden, wodurch der Gesamtstrom, der für den Betrieb eines Arrays mehrerer Laserdiodenelemente 30 benötigt wird, vergleichsweise gering ist. Dieser Zusammenhang ist in Fig. 9a und 9b gut dargestellt. Es ist erkennbar, dass in der Serienschaltung gemäß Fig. 9a ein geringerer Gesamtstrom I_ges benötigt wird, als bei der Parallelschaltung mehrerer Laserdiodenelemente 30 gemäß Fig. 9b.

Ein besonders bevorzugtes Ausführungsbeispiel des erfindungsgemäßen Systems ist in Fig. 8 gezeigt. Das Lasergerät 1 ist bei diesem Ausführungsbeispiel stiftartig ausgebildet und vorzugsweise derart dimensioniert, dass es mit einer Hand des Anwenders bedienbar ist. Im Wesentlichen gleicht das Lasergerät 1 also einem Stift, wobei das Lasergerät 1 entsprechend kompakt ist und so eine einfache und äußerst mobile Anwendung ermöglicht. Der Stift bzw. das Lasergerät 1 weist ein Außengehäuse 10 auf, wobei in das Außengehäuse 10 ein Akkumulator 28 bzw. eine Batterie integriert ist. Der Akkumulator 28 ist mit der Steuerung 13 elektrisch verbunden, die vorzugsweise als Mikrocontroller auf einem Printed Circuit Board (PCB) ausgestaltet sein kann. Die Steuerung 13 umfasst ferner einen Laserdiodentreiber 13b bzw. ist mit einem Laserdiodentreiber 13b gekoppelt. Der Laserdiodentreiber 13b ist wiederum elektrisch mit einem Laserdiodenmodul verbunden, das mehrere Laserdiodenelemente 30 sowie die Koppeloptik 11b aufweisen kann.

Das Lasergerät 1 bzw. der Stift umfasst ferner einen Aktivierungsschalter 29 über welchen die Laserdiodenelemente 30 ein- bzw. ausgeschaltet werden können. Schließlich ist bevorzugt vorgesehen, dass das Lasergerät 1 bzw. der Stift ein Display und Bedienelemente aufweist. Vorzugsweise sind das Display und die Bedienelemente als Touchscreen 14 kombiniert. Das Lasergerät 1 weist ferner eine Schnittstelle, beispielsweise einen USB-Anschluss 39 auf. Der USB-Anschluss 39 kann einerseits zum Datenaustausch und andererseits zur Verbindung des Akkumulators 28 mit einer Stromquelle zum Zwecke der Wiederaufladung des Akkumulators 28 genutzt werden. Hinsichtlich des Datenaustauschs kann insbesondere vorgesehen sein, dass der USB-Anschluss 39 mit einem Fluoreszenzlichtdetektor 36 signalverbindbar ist, um eine Ansteuerung der Laserdiodenelemente 30 anhand von Daten über das zu behandelnde Gewebe zu ermöglichen. Ein solcher Fluoreszenzlichtdetektor 36 kann beispielsweise in eines der Applikationsmodule 200 integriert sein, die in Fig. 8 beispielhaft dargestellt sind. Mit gestrichelter Linie ist in diesem Zusammenhang ein Rückkopplungskanal von einem Fluoreszenzlichtdetektor 36 zum USB-Anschluss 39 symbolisiert.

Das Lasergerät 1 weist außerdem ein Schutzglas 33 auf, dass im Strahlengang des Lasers, vorzugsweise der Einkoppellinse nachfolgend, angeordnet ist. Das Schutzglas 33 ist vorzugsweise auf das Außengehäuse 10 des Lasergeräts 1 aufschraubbar. Dies ermöglicht einen leichten Austausch bzw. Wechsel des Schutzglases 33.

Ferner sind auf das Schutzglas 33 bzw. das Lasergerät 1 mehrere unterschiedliche Applikationsmodule 200 aufsetzbar, insbesondere aufschraubbar. In Fig. 8 sind vier verschiedene Applikationsmodule 200 beispielhaft dargestellt. So kann ein Applikationsmodul 200 vorgesehen sein, welches jeweils eine Austrittsoptik 40 aufweist. Die Austrittsoptik 40 kann ein oder mehrere Linsen umfassen, die den Laserstrahl L fokussieren.

Bei einer erste Variante eines Applikationsmoduls 200 gemäß Fig. 8 ist vorgesehen, dass das Applikationsmodul 200 außerdem einen Abstandshalter 38 aufweist, der dazu dient, einen gleichmäßigen Abstand zwischen dem zu behandelnden Gewebe und dem Applikationsmodul 200 sicherzustellen. Dies ist besonders vorteilhaft, wenn die Austrittsoptik 40 eine feste Brennweite bzw. Fokuslänge aufweist. Alternativ kann vorgesehen sein, dass das Applikationsmodul eine variable Fokuslänge bzw. Brennweite umfasst. Eine solche Alternative ist ebenfalls in Fig. 8 dargestellt, wobei der Doppelpfeil die Verschiebung der Brennweite bzw. Fokuslänge symbolisiert.

Schließlich kann in das Applikationsmodul 200 auch ein Strahlteiler 34 und ein Fluoreszenzlichtdetektor 36 integriert sein, so dass in das Applikationsmodul 200 rückgekoppeltes Fluoreszenzlicht erfasst und an das Lasergerät 1 zur Steuerung der Laserdiodenelemente 30 zurückgesendet werden kann.

Die Austrittsoptik 40 kann außerdem durch eine Einkoppellinse 32 gebildet sein, so dass mittels der Austrittsoptik 40 des Applikationsmoduls 200 das Laserlicht in einen Lichtwellenleiter 25 eingekoppelt werden kann. Dazu ist das Applikationsmodul 200 vorzugsweise mit einem Lichtwellenleiter 25 verbindbar, wobei der Lichtwellenleiter 25 an seinem distalen Ende vorzugsweise ein entsprechendes Anschlussstück, insbesondere einen Faserstecker 26, aufweist. Das in der vorliegenden Anmeldung beschriebene System eignet sich nicht nur zur endoskopischen Chirurgie, insbesondere zur endovenösen Lasertherapie, sondern findet, insbesondere als sogenanntes Laserskalpell, bevorzugt Einsatzmöglichkeiten in der Dermatologie, beispielsweise zur Entfernung von Fibromen bzw. Warzen, im Bereich der Gastroenterologie, der Gefäßchirurgie, der Gynäkologie, der Halsnasenohren-Chirurgie, der Laparoskopie, der Orthopädie, der Pädiatrie, der Pulmologie, der Urologie und/oder der Thorax-Chirurgie. Weitere Anwendungsmöglichkeiten ergeben sich in der Zahnheilkunde oder Veterinärmedizin.

Insbesondere kann das hier beschriebene System modular aufgebaut sein, wobei das Lasergerät 1 das Grundmodul des modularen Systems darstellt. Das Lasergerät 1 ist jedoch mit unterschiedlichen Applikationsmodulen koppelbar, die sich insbesondere in ihrem Aufbau unterscheiden. Insbesondere Applikationsmodule, die mit jeweils wenigstens einem Lichtwellenleiter ausgestattet sind, können sich durch die unterschiedlichen Gestaltungen des Lichtwellenleiters, insbesondere im Hinblick auf die distalen Enden des Lichtwellenleiters, unterscheiden.

### Bezugszeichenliste

- 1: Lasergerät
- 10: Außengehäuse
- 11: Laserlichtquelle
- 11a: Kühlkörper
- 11b: Koppeloptik
- 12: Modulanschluss
- 13: Steuerung
- 13a: Hauptsteuerung
- 13b: Laserdiodentreiber
- 13c: Verstärker
- 13d: Pulsgenerator
- 14: Touchscreen
- 15: Schlüsselschalter
- 16: Leuchtanzeige
- 17: Innengehäuse
- 18: Lüftereinheit
- 19: Netzteil
- 20: Katheter
- 21: Fußschalter
- 22: Fußschalteranschluss
- 23: NotausSchalter
- 24: Hauptschalter
- 25: Lichtwellenleiter
- 25a: Faser
- 25b: distales Faserende
- 25c: Reflektor
- 26: Faserstecker
- 27: RFID-Chip
- 28: Akkumulator
- 29: Aktivierungsschalter
- 30: Laserdiodenelement
- 31: Kollimationslinse
- 32: Einkoppellinse
- 33: Schutzglas
- 34: Strahlteiler
- 34a: erster Strahlteiler
- 34b: zweiter Strahlteiler
- 35: Hilfslaserlichtquelle
- 36: Fluoreszenzlichtdetektor
- 37: optischer Filter
- 38: Abstandshalter
- 39: USB-Anschluss
- 40: Austrittsoptik
- 41: Hilfslaserlichtdetektor
- 200: Applikationsmodul
- 300: Laserdiodenmodul
- L: Laserlicht
- AR: Absorptionsrate
- TR: Transmissionsrate

## Patentansprüche

1. System zur chirurgischen Behandlung mit einem Lasergerät (1) und einem Applikationsmodul (200), wobei das Lasergerät (1) eine Laserlichtquelle (11) mit mehreren Laserdiodenelementen mit unterschiedlicher Wellenlänge aufweist und das Applikationsmodul (200) mit der Laserlichtquelle (11) optisch verbindbar oder verbunden ist, wobei das erste Laserdiodenelement wenigstens eine Halbleiterschicht aus einer Antimon-Verbindung aufweist und so konfiguriert ist, dass Laserlicht mit einer ersten Wellenlänge zwischen 1800 nm und 2000 nm erzeugbar ist,
wobei das Applikationsmodul (200) als biegeflexibler Katheter (20) mit einem Lichtwellenleiter ausgebildet ist, der einen RFID-Chip mit einer Parameter- und/oder Freigabecodierung aufweist, wobei das Lasergerät (1) eine Steuerung (13) mit einer RFID-Sender- und Empfängereinheit zum Auslesen und Beschreiben des RFID-Chips aufweist und die Steuerung (13) derart konfiguriert ist, dass
- eine Aktivierung der Laserlichtquelle (11) in Abhängigkeit von der Erfassung einer vorbestimmten Parameter- und/oder Freigabecodierung durch die RFID-Empfängereinheit erfolgt, und
- zur Entwertung des Katheters ein Zeitstempel auf dem RFID-Chip gespeichert wird.

2. System nach Anspruch 1,
**dadurch gekennzeichnet, dass**
die Halbleiterschicht eine Galliumantimonid-Verbindung aufweist oder daraus besteht.

3. System nach Anspruch 1 oder 2,
**dadurch gekennzeichnet, dass**
mehrere erste Laserdiodenelemente zu einem Array mit einer hexagonalen Linsenstruktur verschaltet sind.

4. System nach einem der vorhergehenden Ansprüche,
**dadurch gekennzeichnet, dass**
die Laserlichtquelle (11) wenigstens ein zweites Laserdiodenelement aufweist, das Laserlicht mit einer zweiten Wellenlänge emittiert, die sich von der ersten Wellenlänge unterscheidet.

5. System nach einem der vorhergehenden Ansprüche,
**dadurch gekennzeichnet, dass**
das Lasergerät (1) und/oder das Applikationsmodul (200) eine Koppeloptik (11b) zur Einkopplung des von der Laserlichtquelle (11) erzeugten Laserlichts in den Lichtwellenleiter aufweist.

6. System nach Anspruch 4 und 5,
**dadurch gekennzeichnet, dass**
das erste Laserdiodenelement und das zweite Laserdiodenelement derart angeordnet sind, dass von dem ersten und dem zweiten Laserdiodenelement emittiertes Laserlicht abwechselnd und/oder gleichzeitig in den Lichtwellenleiter einkoppelbar ist.

7. System nach Anspruch 5 oder 6,
**dadurch gekennzeichnet, dass**
mehrere erste Laserdiodenelemente und/oder mehrere zweite Laserdiodenelemente in elektrischer Serienschaltung miteinander verbunden sind.

8. System nach einem der vorhergehenden Ansprüche,
**dadurch gekennzeichnet, dass**
die Steuerung (13) mit der Laserlichtquelle (11) verbunden und so konfiguriert ist, dass eine gepulste und/oder kontinuierliche Laserlichtemission einstellbar ist.

9. System nach einem der vorhergehenden Ansprüche,
**dadurch gekennzeichnet, dass**
die Laserlichtquelle (11) mit einer elektrischen Betriebsgleichspannung von höchstens 50 Volt betreibbar ist.

10. System nach einem der vorhergehenden Ansprüche,
**dadurch gekennzeichnet, dass**
die Laserlichtquelle (11) ein gas- und/oder flüssigkeitsdichtes Innengehäuse (17) aufweist.

11. System nach einem der vorhergehenden Ansprüche,
**dadurch gekennzeichnet, dass**
das Lasergerät (1) eine Hilfslaserlichtquelle und einen Lichtsensor umfasst, wobei die Hilfslaserlichtquelle und der Lichtsensor mit der Steuerung (13) derart gekoppelt sind, dass eine Laserlichtintensität der Laserlichtquelle (11) in Abhängigkeit einer spezifischen Fluoreszenzlichtsignatur des zu behandelnden Gewebes einstellbar ist.

12. System nach einem der vorhergehenden Ansprüche,
**dadurch gekennzeichnet, dass**
das Lasergerät (1) einen Temperatursensor zur Erfassung einer Temperatur des zu behandelnden Gewebes aufweist, der mit der Steuerung (13) derart gekoppelt ist, dass eine Laserlichtintensität der Laserlichtquelle (11) in Abhängigkeit der Gewebetemperatur einstellbar ist.

13. System nach einem der vorhergehenden Ansprüche,
**dadurch gekennzeichnet, dass**
das Lasergerät (1) eine Speichereinheit zur Speicherung von Behandlungsinformationen aufweist.

14. System nach einem der vorhergehenden Ansprüche,
**dadurch gekennzeichnet, dass**
das Lasergerät (1) mit unterschiedlichen Applikationsmodulen (200) verbindbar ist.

15. System nach einem der vorhergehenden Ansprüche,
**dadurch gekennzeichnet, dass**
das Lasergerät (1) derart kompakt und leicht ausgebildet ist, dass das Lasergerät (1) stiftartig mit einer Hand geführt werden kann.

## Claims

1. A system for surgical treatment comprising a laser device (1) and an application module (200), wherein the laser device (1) comprises a laser light source (11) having a plurality of laser diode elements with different wavelengths and the application module (200) is optically connectable or connected to the laser light source (11), wherein the first laser diode element comprises at least one semiconductor layer from an antimonide compound and is configured such that laser light can be generated at a first wavelength between 1800 nm and 2000 nm, wherein the application module (200) is designed as a flexurally flexible catheter (20) having an optical waveguide comprising an RFID chip with a parameter and/or release coding, wherein the laser device (1) comprises a controlling means (13) with an RFID transmitter and receiver unit for reading from and writing to the RFID chip and the controlling means (13) is configured such that
- an activation of the laser light source (11) ensues in response to the RFID receiver unit detecting a predetermined parameter and/or release coding, and
- a timestamp is stored on the RFID chip for the invalidating of the catheter.

2. The system according to claim 1,
**characterized in that**
the semiconductor layer comprises or consists of a gallium antimonide compound.

3. The system according to claim 1 or 2,
**characterized in that**
a plurality of first laser diode elements are connected into an array having a hexagonal lens structure.

4. The system according to one of the preceding claims, **characterized in that**
the laser light source (11) comprises at least one second laser diode element which emits laser light at a second wavelength differing from the first wavelength.

5. The system according to one of the preceding claims, **characterized in that**
the laser device (1) and/or the application module (200) comprises a coupling optic (11b) for coupling the laser light produced by the laser light source (11) into the optical waveguide.

6. The system according to claim 4 and 5,
**characterized in that**
the first laser diode element and the second laser diode element are arranged such that laser light emitted from the first and second laser diode element can be alternatingly and/or concurrently coupled into the optical waveguide.

7. The system according to claim 5 or 6,
**characterized in that**
a plurality of first laser diode elements and/or a plurality of second laser diode elements are connected together in electrical series connection.

8. The system according to one of the preceding claims, **characterized in that**
the controlling means (13) is connected to the laser light source (11) and
configured such that a pulsed and/or continuous laser light emission can be regulated.

9. The system according to one of the preceding claims,
**characterized in that**
the laser light source (11) is operable at an electrical operating DC voltage of not more than 50 volts.

10. The system according to one of the preceding claims,
**characterized in that**
the laser light source (11) comprises a gas-tight and/or liquid-tight interior housing (17).

11. The system according to one of the preceding claims,
**characterized in that**
the laser device (1) comprises an auxiliary laser light source and a photosensor, wherein the auxiliary laser light source and the photosensor are coupled to the controlling means (13) such that a laser light intensity of the laser light source (11) can be adjusted as a function of a specific fluorescent light signature of the tissue to be treated.

12. The system according to one of the preceding claims,
**characterized in that**
the laser device (1) comprises a temperature sensor for detecting a temperature of the tissue to be treated, same being coupled to the controlling means (13) such that a laser light intensity of the laser light source (11) can be adjusted as a function of the tissue temperature.

13. The system according to one of the preceding claims,
**characterized in that**
the laser device (1) comprises a storage unit for storing treatment information.

14. The system according to one of the preceding claims,
**characterized in that**
the laser device (1) can be connected to different application modules (200).

15. The system according to one of the preceding claims,
**characterized in that**
the laser device (1) is of such compact and lightweight construction that the laser device (1) can be controlled with one hand like a pen.

## Revendications

1. Système pour traitement chirurgical avec un appareil laser (1) et avec un module d'application (200), dans lequel l'appareil laser (1) comprend une source de lumière laser (11) avec plusieurs éléments de diodes laser avec des longueurs d'ondes différentes et le module d'application (200) est relié ou susceptible d'être relié optiquement avec la source de lumière laser (11), dans lequel le premier élément de diode laser comprend au moins une couche semiconductrice d'un composé à base d'antimoine et est ainsi configuré que la lumière laser peut être engendrée avec une première longueur d'onde entre 1800 nm et de 1000 nm,
dans lequel le module d'application (200) est réalisé sous forme de cathéter flexible souple (20) avec un guide d'onde de lumière qui comprend une puce RFID avec un codage paramétrique et/ou un codage d'autorisation, dans lequel l'appareil laser comprend une commande (13) avec une unité émettrice/réceptrice à RFID pour la lecture et l'écriture de la puce RFID, et la commande (13) est ainsi configurée que
- une activation de la source de lumière laser (11) a lieu en dépendance de la détection, par l'unité réceptrice RFID, d'un codage paramétrique et/ou d'un codage d'autorisation prédéterminé, et
- pour l'invalidation du cathéter un marquage horodateur est mémorisé sur la puce RFID.

2. Système selon la revendication 1,
**caractérisé en ce que** la couche semiconductrice comprend un composé gallium-antimoine ou est constituée d'un tel composé.

3. Système selon la revendication 1 ou 2,
**caractérisé en ce que** plusieurs premiers éléments de diodes laser sont mis en circuits pour former un réseau avec une structure de lentille hexagonale.

4. Système selon l'une des revendications précédentes,
**caractérisé en ce que** la source de lumière laser (11) comprend au moins un second élément de diode laser, qui émet de la lumière laser avec une seconde longueur d'onde, qui diffère de la première longueur d'onde.

5. Système selon l'une des revendications précédentes,
**caractérisé en ce que** l'appareil laser (1) et/ou le module d'application (200) comprend une optique de couplage (11b) pour le couplage entrant de la lumière laser engendrée par la source de lumière laser (11).

6. Système selon la revendication 4 ou 5,
**caractérisé en ce que** le premier élément de diode laser et le second élément de diode laser sont agencés de telle façon que la lumière laser émise par le premier et par le second élément de diode laser est susceptible d'être couplée en entrant dans le guide d'onde de lumière en alternance et/ou simultanément.

7. Système selon la revendication 5 ou 6,
**caractérisé en ce que** plusieurs premiers éléments de diodes laser et/ou plusieurs seconds éléments de diodes laser sont reliés les uns aux autres dans un circuit électrique en série.

8. Système selon l'une des revendications précédentes,
**caractérisé en ce que** la commande (13) est reliée à la source de lumière laser (11) et est ainsi configurée qu'il est possible d'établir une émission de lumière laser pulsée et/ou continue.

9. Système selon l'une des revendications précédentes,
**caractérisé en ce que** la source de lumière laser (11) peut être amenée à fonctionner avec une tension de service électrique continue de 50 V au maximum.

10. Système selon l'une des revendications précédentes,
**caractérisé en ce que** la source de lumière laser (11) comprend un boîtier intérieur (17) étanche aux gaz et/ou étanche aux liquides.

11. Système selon l'une des revendications précédentes,
**caractérisé en ce que** l'appareil laser (1) inclut une source de lumière laser auxiliaire et un capteur de lumière, dans lequel la source de lumière laser auxiliaire et le capteur de lumière sont couplés avec la commande (13) de telle manière qu'il est possible d'établir une intensité de lumière laser de la source de lumière laser (11) en fonction d'une signature spécifique de la lumière par fluorescence du tissu qu'il s'agit de traiter.

12. Système selon l'une des revendications précédentes,
**caractérisé en ce que** l'appareil laser (1) comprend un capteur de température pour détecter une température du tissu qu'il s'agit de traiter, ledit capteur étant couplé avec la commande (13) de telle façon qu'il est possible d'établir une intensité de lumière laser de la source de lumière laser (11) en fonction de la température du tissu.

13. Système selon l'une des revendications précédentes,
**caractérisé en ce que** l'appareil laser (1) comprend une unité à mémoire pour la mémorisation d'informations relatives au traitement.

14. Système selon l'une des revendications précédentes,
**caractérisé en ce que** l'appareil laser (1) est susceptible d'être relié à différents modules d'application (200).

15. Système selon l'une des revendications précédentes,
**caractérisé en ce que** l'appareil laser (1) est réalisé de manière compactée légère, de telle façon que l'appareil laser (1) peut être guidé avec une main à la manière d'un stylet.
